# EUROPEAN PATENT SPECIFICATION

(11) **EP 4 021 312 B1**
(45) Date of publication and mention of the grant of the patent: **18.02.2026**
(21) Application number: 20839497.3
(22) Date of filing: 16.12.2020
(51) Int. Cl.: A61B 17/00, A61M 37/00

(54) **AGENT DELIVERY DEVICE**
WIRKSTOFFABGABEVORRICHTUNG
DISPOSITIF D'ADMINISTRATION D'AGENT

(30) Priority: 20.12.2019 US 201962951426 P
(43) Date of publication of application: 06.07.2022
(73) Proprietor: Boston Scientific Scimed, Inc., Maple Grove, MN 55311-1566 (US)
(72) Inventor: CONGDON, Daniel, Hudson, MA 01749 (US); JAGELSKI, Matthew, Robert, Marlborough, MA 01752 (US); LEHTINEN, Laurie, A., Boylston, MA 01505 (US); PIC, Andrew, Northboro, MA 01532 (US)
(74) Representative: Vossius & Partner Patentanwälte Rechtsanwälte mbB
(86) International application number: PCT/US2020/065271
(87) International publication number: WO 2021/126951

(56) References cited:
- US-A- 4 762 515
- US-A1- 2006 247 578
- US-A1- 2008 103 564
- US-A1- 2016 220 482
- US-A1- 2019 232 030

## Description

### Technical Field

The present disclosure relates generally to a medical device that administers an agent. More particularly, at least some embodiments of the present disclosure relate to a medical device configured to be loaded with a therapeutic agent, separate the loaded agent into a smaller form, and then deliver that agent via a lumen of the medical device.

### Background

**In** certain medical procedures, it may be necessary to stop bleeding internal to the body. For example, an endoscopic medical procedure may require hemostasis of bleeding tissue within the gastrointestinal tract, for example in the esophagus, stomach, or intestines.

During an endoscopic procedure, a user inserts a sheath of an endoscope into a body lumen of a patient. The user utilizes a handle of the endoscope to control the endoscope during the procedure. Tools are passed through a working channel of the endoscope via, for example, a port in the handle, to deliver treatment at the procedure site near a distal end of the endoscope. The procedure site is remote from the operator.

To achieve hemostasis at the remote site, a hemostatic agent may be delivered by a device inserted into the working channel of the endoscope. Agent delivery may be achieved through mechanical systems, for example. Such systems, however, may require numerous steps or actuations to achieve delivery, may not achieve a desired rate of agent delivery or a desired dosage of agent, may result in the agent clogging portions of the delivery device, may result in inconsistent dosing of agent, or may not result in the agent reaching the treatment site deep within the GI tract.
US 2019/0232030 A1 discloses an apparatus for delivering a powdered agent into a subject's body which may include a first passage for receiving a pressurized gas. The apparatus also may include a container housing a powdered agent. The container may be in fluid connection with the first passage. At least a portion of the pressurized gas is introduced into the powdered agent in the container to fluidize the powdered agent. The apparatus also may include a second passage for receiving the powdered agent from the container. In a first configuration of the apparatus, the second passage may not be in fluid connection with the container. In a second configuration of the apparatus, the second passage may be in fluid connection with the container. The apparatus may be configured to transition between the first configuration and the second configuration.

US 2016/220482 A1 discloses an apparatus for delivering a therapeutic agent. The apparatus comprises a carriage for holding and dispensing a plurality of medication pellets, each pellet containing a selected dose of drug to treat a medical condition. A catheter is positioned in the apparatus, having a lumen sized for the pellet with a distal end for delivering the pellet to a delivery site. A pusher plate can engage and advance the pellet through the catheter to the delivery site. Alternatively, pressurized liquid can be used to advance the pellet out of the catheter. Prior to or after delivery, the pellet can be treated with mechanical, electromagnetic, acoustical or other energy to weaken the pellet structure, create cracks for the ingress of fluids or initiate the breakup of the pellet into smaller pieces. This can be achieved from a mechanical compressive force applied to the pellet prior to delivery.

The current disclosure may solve one or more of these issues or other issues in the art.

### Summary of the Disclosure

The claimed invention comprises a medical device as defined by the appended independent claim 1. Further embodiments of the claimed medical device are described in the appended dependent claims.

Also disclosed is a method of administering agent via a medical device which may include positioning a lumen of the medical device so that a distal end of the lumen is adjacent to a targeted site, wherein the device further includes a housing defining at least one enclosure storing the agent in a first form, a force applicator within the housing and adjacent the enclosure, and a drive mechanism for moving the agent toward the force applicator, providing a pressurized fluid to the lumen, and delivering the agent towards the force applicator via the drive mechanism, thereby separating the agent into particles smaller than a size of the first form via the force applicator, and feeding the lumen with the particles.

### Brief Description Of The Drawings

The accompanying drawings, which are incorporated in and constitute a part of this specification, illustrate various exemplary embodiments and together with the description, serve to explain the principles of the disclosed embodiments.
FIGS. 1A-1B are cross-sectional views of a medical device.
FIG. 2 is a cross-sectional view of an enclosure and a feeding mechanism of a medical device.
FIG. 3 is a cross-sectional view of a portion of a medical device.
FIG. 4A is a cross-sectional view of a medical device.
FIG. 4B is a top view of a holster of the medical device of FIG. 4A.
FIGS. 4C and 4D are cross-sectional views of a medical device.

### Detailed Description

Reference will now be made in detail to aspects of the present disclosure and of the claimed invention, examples of which are illustrated in the accompanying drawings. Wherever possible, the same or similar reference numbers will be used through the drawings to refer to the same or like parts. The term "distal" refers to a portion farthest away from a user when introducing a device into a subject (e.g., patient). By contrast, the term "proximal" refers to a portion closest to the user when placing the device into the subject.

Both the foregoing general description and the following detailed description are exemplary and explanatory only and are not restrictive of the features. As used herein, the terms "comprises," "comprising," "having," "including," or other variations thereof, are intended to cover a non-exclusive inclusion such that a process, method, article, or apparatus that comprises a list of elements does not include only those elements, but may include other elements not expressly listed or inherent to such a process, method, article, or apparatus. **In** this disclosure, relative terms, such as, for example, "about," "substantially," "generally," and "approximately" are used to indicate a possible variation of ±10% in a stated value or characteristic.

The present disclosure may solve one or more of the limitations in the art. The scope of the claimed invention, however, is defined by the attached claims and not the ability to solve a specific problem. The present disclosure is drawn to medical devices configured to be loaded with agent(s), e.g., therapeutic agents, that crush or separate the loaded agent(s) into smaller particles, and administer said particles to a targeted site, among other aspects. The agent may be in any first form, e.g., a rod, a pellet, prior to it being separated or crushed into a smaller form, such as a powder of loose particles, and be delivered to a lumen receiving a stream of propellant/pressurized fluid, e.g., CO₂, nitrogen, air, etc. Said medical devices may help increase the consistency of particle size and particle delivery of agent, e.g., hemostatic powder, and may also help reduce variation that is inherent in conventional fluid-driven powder/particle mixing and delivery systems.

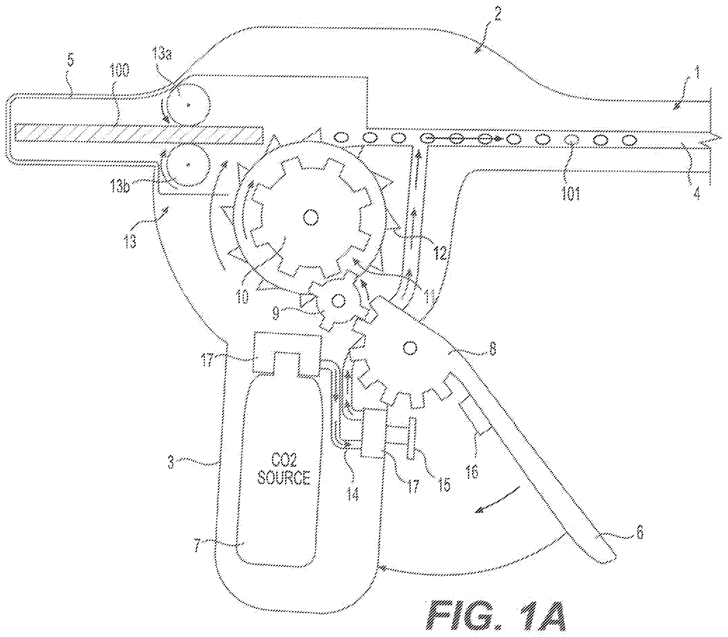

FIG. 1A illustrates a medical device 1 in further detail. Medical device 1 includes a housing 2 defining at least one enclosure 5 for storing agent 100 in a first form, e.g., a rod or other single-piece shaped form of an agent, a force applicator 11 within housing 2 and adjacent enclosure 5, and a drive mechanism 13 configured for moving or propelling agent 100 towards force applicator 11. Enclosure 5 may be pre-loaded with agent 100, or enclosure 5 may include an opening/mechanism by which it may be loaded with agent 100. Force applicator 11 defines a surface, e.g., a surface of each of a plurality of teeth 12 along its circumference, for applying a force to agent 100 to separate it into particles 101 smaller than its first form. Instead of teeth 12 about its circumference, force applicator 11 could include serrations, barbs, or any other sharp or roughened surface capable of separating agent into powder/particle form. Drive mechanism 13 includes two wheels 13a and 13b positioned directly below and above one another, with sufficient space between one another to receive agent 100. Drive mechanism 13 may be within enclosure 5, in which agent 100 is stored. Drive mechanism 13 moves agent 100 towards force applicator 11 as wheel 13a rotates counter-clockwise and wheel 13b rotates clockwise. However, drive mechanism 13 is not limited to wheels 13a and 13b, and may be any suitable mechanism for advancing agent 100. Similarly, force applicator 11 may be any suitable mechanism for separating/crushing agent 100 into particles 101, such as, round grinders, worm gears, or augers. In some other embodiments, device 1 may include a plurality of force applicators.

Medical device 1 also includes a lumen 4 within housing 2 for receiving particles 101 from force applicator 11 and for receiving a pressurized fluid from a fluid source 7, via a channel 14, to propel particles 101 through lumen 4. Lumen 4 may be connected with or otherwise be in fluid communication with enclosure 5 storing agent 100, so that a distal portion of enclosure 5 transitions into lumen 4, as shown in FIG. 1A. Channel 14 includes a valve 17 along its length between source 7 and lumen 4, and is connected to a portion of lumen 4 that is distal to force applicator 11 to feed lumen 4 with pressurized fluid, thereby propelling particles 101 towards a distal end of lumen 4. Valve 14 is coupled to a trigger 15 located outside of a handle portion 3 of housing 2. Trigger 15 may be configured to at least open fluid valve 14, thereby providing a flow of pressurized fluid from fluid source 7 to lumen 4. Trigger 15 may be any suitable form, e.g., a button, a switch, and its form is not particularly limited. Fluid source 7 is within handle portion 3 of housing 2, but is not particularly limited to being within handle 3, and may even be outside housing 2. It is further noted that lumen 4 is not limited to being within housing 2, and in other embodiments, may be at least partially outside of housing 2. A catheter/sheath (not shown) may also be attached to (or otherwise extend from) the distal end of housing 2. Said catheter/sheath may be long, flexible to traverse tortuous patient anatomy, and any suitable size to insert into a working lumen of a scope (not shown) or another delivery device (not shown).

Medical device 1, shown in FIG. 1A, further includes a first gear 10 that is coupled to a surface of force applicator 11, and first gear 10 is configured to rotate simultaneously with force applicator 11, in the same direction. Medical device 1 also includes a lever 6 coupled to housing 2, and the coupled end of lever 6 includes a second gear 8. First gear 10 and second gear 8 are connected in series via a linking gear 9, positioned in between first gear 10 and second gear 8. Thus, lever 6 is pivotably coupled to linking gear 9, via second gear 8. As a result of such configuration, pulling lever 6 proximally rotates second gear 8 (clockwise as shown in FIG. 1A), which in turn rotates linking gear 9 (counter-clockwise), which rotates first gear 10 (clockwise) by a selected or desired degree. Pulling lever 6 proximally may entail translating one end of lever 6 (the end opposite of second gear 8) towards handle portion 3, as indicated by the directional arrow of FIG. 1A. This translation is a pivoted movement, as the other end of lever 6 (second gear 8) is pivotably connected to linking gear 9. The pulling of lever 6 may be by any suitable action, for example, by hand or by mechanical, electrical, or pneumatic action. The rotation of first gear 10 rotates force applicator 11 by a selected or desired degree, which proceeds to separate/crush a portion of agent 100, fed via drive wheels 13a and 13b, into particles 101. It is noted that the rotation of drive wheels 13a and 13b may be actuated via any suitable mechanism. Such mechanisms may include an automated mechanism that is triggered by the pulling of lever 6 or by the actuation of trigger 15 opening valve 17. Another mechanism may include an additional gear or a series of gears connecting first gear 10 to drive wheels 13a and 13b, and thus, also rotating said drive wheels by pulling lever 6.

Lever 6 further includes a contact 16, which may be a protrusion or a tab extending outward from the surface of lever 6 facing handle 3. Contact 16 may be configured to press or actuate trigger 15 on handle 3, as lever 6 is pulled proximally towards handle 3, thereby opening valve 17. Opening valve 17 permits pressurized fluid to flow into channel 14 past valve 17 and into lumen 4 to propel particles 101 distally. Referring to FIG. 1A, an example of how medical device 1 may be used is further discussed below. The distal portion of medical device 1 (e.g., a catheter or sheath having the distal portion of lumen 4) may be delivered into the body of a subject. Lumen 4 may be positioned so that a distal end of lumen 4 is adjacent to an intended target site for agent 100 administration. Such delivery and positioning may be accomplished via an endoscope having a working channel (not shown). Imaging associated with the endoscope may assist in positioning. A user may then load housing 2 of medical device 1 with agent 100, if not loaded already, so that wheels 13a and 13b of driving mechanism 13 may advance agent 100 towards force applicator 11. The manner in which housing 2 is loaded with agent 100 is not particularly limited. A user may then pull lever 6 proximally, by any suitable manner/ mechanism, so that contact 16 presses against trigger 15, thereby opening valve 14 and providing a pressurized fluid to lumen 4, and also rotating force applicator 11 via the series of first gear 10, linking gear 9, and second gear 8. This results in force applicator 11 separating/crushing agent 100 into particles 101, via the force applied by teeth 12 to agent 100. Lumen 4 is fed with particles 101, which are propelled toward the distal end of lumen 4 via pressurized fluid and, thus, administered to the intended target site. Pulling lever 6 may also trigger a mechanism by which driving wheels 13a and 13b are rotated, such as via automated rotation or via a gear or a series of additional gears connecting first gear 10 to driving wheels 13a and 13b.

It is further noted that a throw of lever 6 rotates force applicator 11 by a certain degree, as discussed above, and supplies a continuous flow of pressurized fluid, as long as trigger 15 is pressed inward. Thus, a single throw of lever 6 may deliver a consistent, desired dose of agent 100, separated into particles 101, into lumen 4. For example, FIG. 1A shows that lever 6 may be rotated about 45 degrees clockwise, causing a set degree of rotation of gears 9, 10 and force applicator 11. This set amount of rotation corresponds to a set amount of agent 100 being separated into particles. For larger doses, a user may repeatedly actuate (push then pull) lever 6 so that force applicator 11 is intermittently rotated and pressurized fluid is supplied. Alternatively, for larger doses, the throw of lever 6 may be increased.

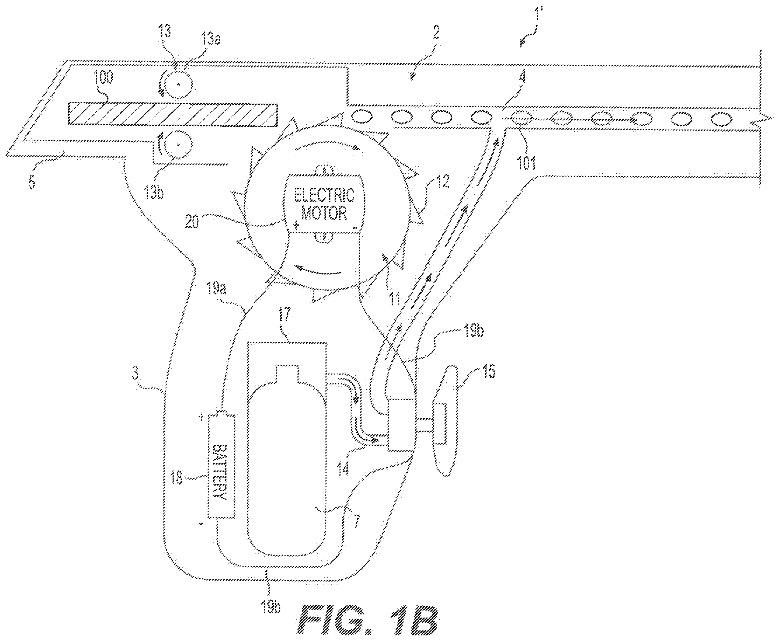

Medical device 1', as shown in FIG. 1B, is similar to device 1 in many respects. Like reference numeral refer to like parts. Differences between device 1 and 1' will be described. Device 1' includes an electric motor 20 coupled to force applicator 11, configured to rotate force applicator 11 electrically. In some other embodiments, medical device 1' may include a plurality of electric motors 20 and/or a plurality of force applicators 11. Medical device 1' further includes a battery 18 electrically connected to electric motor 20, via wires 19a and 19b, to power electric motor 20. Wire 19a connects the cathode of battery 18 to electric motor 20. Wire 19b includes two portions to connect the anode of battery 18 and motor 20 to trigger 15', which serves as both a valve switch and an electrical switch, along its path to electric motor 18. Therefore, the actuation of trigger 15' may open valve 17 and power electric motor 20 simultaneously in medical device 1'. The actuation of trigger 15' may be by any suitable action, for example, by hand or by mechanical, electrical, or pneumatic action.

Medical device 1' may be used in the same manner as medical device 1 except a user actuates trigger 15', as opposed to pulling a lever. The actuation of trigger 15' opens valve 14, thereby providing a pressurized fluid to lumen 4, and also powers the rotation of force applicator 11 via electric motor 20, which is electrically wired to battery 18. Additionally, the actuation of trigger 15' may also automate the rotation of driving wheels 13a and 13b, thereby feeding agent 100 to force applicator 11. Actuation of trigger 15' may operate the aforementioned functions in a continuous manner, until trigger 15' is released. Thus, a user may hold trigger 15' until a desired amount of agent 100, separated into particles 101, is delivered to a targeted site via lumen 4, and then release trigger 15' to cease the operation of device 1'.

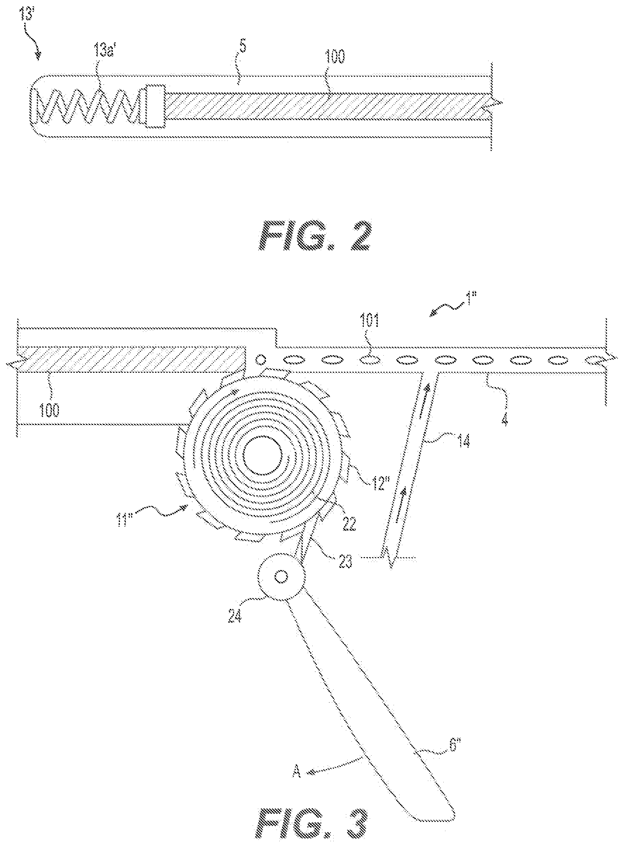

FIG. 2 shows another drive mechanism 13'. Drive mechanism 13' includes a compressed spring 13a' coupled to a platform on one end, that is positioned adjacent to a proximal end of agent 100. The platform defines a surface which pushes against agent 100, as compressed spring 13a' decompresses, thereby pushing agent 100 towards a force applicator (not shown). The other end of spring 13a' is stationary against an inner surface of enclosure 5 in the housing. Agent 100 may be pre-loaded into enclosure 5, or enclosure 5 may include an opening/mechanism by which it may be loaded with agent 100. In this embodiment, a surface of one of the teeth, or any other force applying surface, of a force applicator (not shown) may apply an opposite, greater force against spring 13a', so that spring 13a' remains compressed and agent 100 is not advanced. By such configuration, spring 13a' extends and agent 100 is advanced only when the force applicator is rotated or actuated.

FIG. 3 shows another means by which force applicator 11 may be rotated in medical device 1" to apply a force separating/crushing agent 100 into particles 101. In medical device 1", a torsion spring 22 is coupled to force applicator 11 in a manner so that a torque or a rotary force actuates the rotation of force applicator 11". Medical device 1" further includes a lever 6" configured to pivot about a pivot point 24. Lever 6" includes a pawl 23 that may catch one of teeth 12" of force applicator 11", thereby inhibiting the rotation of force applicator 11". As lever 6" is pulled proximally (as shown by the arrow A), and pivots clockwise about pivot point 24, pawl 23 simultaneously rotates in a clockwise direction, and releases from one of teeth 12" of force applicator 11", thereby rotating force applicator 11" via the rotary force exerted by spring 22. As shown, the rotation of force applicator 11" applies a force to agent 100 via teeth 12", and separates/crushes agent 100 into particles 101, which are subsequently delivered to lumen 4. Thus, medical device 1" may be used in a similar manner as medical device 1. Medical device 1" may also be different in use than device 1. For example, lever 6" may be pulled (as shown by arrow A) and held in its pulled position to permit continuous rotation of force applicator 11', via the rotary force exerted by spring 22, and to have a constant supply of pressurized fluid. Thus, unlike device 1, multiple, sequential throws of lever 6" is not necessary to continually rotate force applicator 1" and supply pressurized fluid for a prolonged duration of time. Lever 6" may also be returned to its original position so that pawl 23 re-engages one of teeth 12" to inhibit further rotation of force applicator 11", and to also cease the supply of pressurized fluid to lumen 4. Lever 6" may be actuated by any suitable action, for example, by hand or by mechanical, electrical, or pneumatic action.

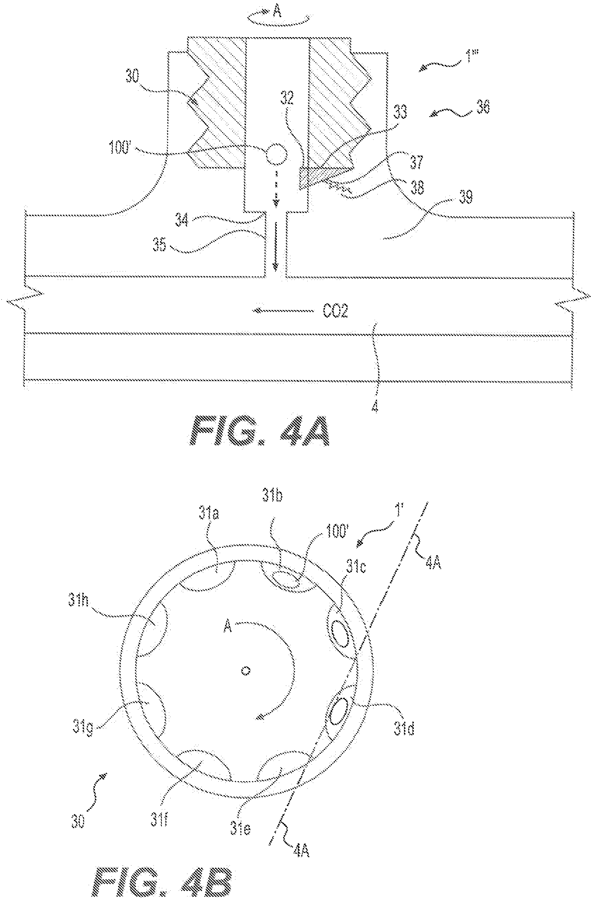

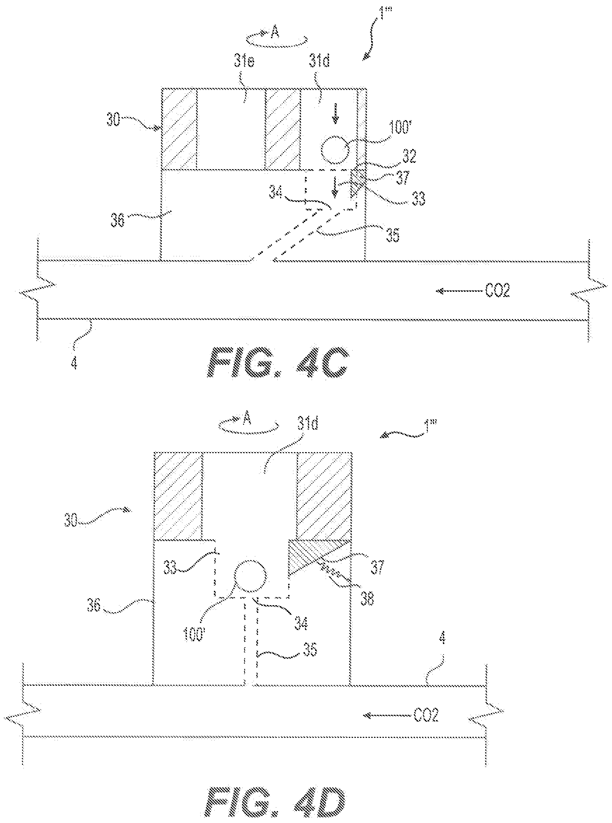

Referring to FIGS. 4A-4D, a medical device 1"' according to the invention is described below. Medical device 1"' includes a housing 36 that includes a holster 30, which includes a plurality of cavities 31a-h for storing agent 100' in a first form, e.g., a pellet. Holster 30 sits within a enclosure of housing 36. FIG. 4A shows a cross-sectional view of holster 30 along line 4A - 4A of FIG. 4B. Medical device 1"' also includes a lumen 4 receiving pressurized fluid, e.g., CO₂, from a fluid source (not shown) at its proximal end. Housing 36 includes a barrier region 39 positioned between holster 30 and lumen 4. As indicated by the directional arrow A in FIGS. 4A and 4B, holster 30 is rotatable relative to a remainder of housing 36 and lumen 4. Furthermore, housing 36 includes a force applicator in the form of a wedge 37, which defines a surface for applying a force to agent 100 to separate it into particles smaller than its first form. The form of a force applicator is not particularly limited to wedge 37, and may be any suitable form. Wedge 37 may be below holster 30, and may be spring-actuated, via spring 38. Any other form of biasing or pressing wedge 37 to the left in FIG. 1A may be used. In another embodiment, wedge 37 may be actuated via pneumatics. An additional port or channel (not shown) is branched from lumen 4 at a point that is proximal to a channel 35, so that said port may feed pressurized fluid directly into housing 36 or specifically towards wedge 37. The force of the fed pressurized fluid may engage wedge 37 to compress and crush agent 100'. In other embodiments, a combination of both a spring and pneumatics may be implemented to actuate wedge 37.

Housing 36 also includes a chamber 33 defined by a first opening 32 that is adjacent to holster 30, and a narrower, second opening 34 leading to a channel 35, which leads to lumen 4. First opening 32 may be aligned with any of the plurality of enclosures 31a-h of holster 30, depending on the rotational position of holster 30 relative to barrier 39. Thus, as holster 30 rotates, agent 100' may drop into chamber 33 from one of the plurality of enclosures 31a-h. The rotation of holster 30 may be by any suitable action, for example, by hand or by mechanical, electrical, or pneumatic action. For example, in some other embodiments, the rotation of holster 30 may be operated by a trigger that causes rotation or measured rotation of holster 30, e.g., rotation such that adjacent enclosures 31a-31h may be aligned with chamber 33 sequentially. **In** FIGS. 4A-4B, holster 30 includes eight enclosures 31a-h distributed evenly about the perimeter/circumference of holster 30. Actuation of a trigger may cause rotation of holster 30 by 45 degrees to align a subsequent enclosure with opening 32. Although eight equally sized and spaced enclosures are shown, it is understood that there may be more or less number of enclosures, varied spacing, and varied size to accommodate different sizes/doses of agent 100'.

A portion of wedge 37, which may be spring-actuated via spring 38, obtrudes the enclosure defined by chamber 33, and after holster 30 releases agent 100' into chamber 33, wedge 37 separates/crushes agent 100' into particles (not shown). Because there is fluid communication between chamber 33 and lumen 4, via channel 35, the particles of agent 100' are delivered to lumen 4, and are propelled towards a distal end of lumen 4 via pressurized fluid. It is noted that agent 100', prior to being separated into particles, is inhibited from falling into channel 35 and being delivered to lumen 4 because second opening 34 and channel 35 are narrower than a width, or cross-sectional size, of agent 100', in whichever first form. A size of opening 34 and channel 35, and a force exerted by wedge 37, may control the size of particles delivered.

Referring to FIGS. 4A-4D, an example of how medical device 1"' may be used is further discussed below. Similar to the aforementioned exemplary medical devices, a distal portion of medical device 1"' (e.g., a catheter or sheath having the distal portion of lumen 4) may be delivered into the body of a subject. Lumen 4 may be positioned/directed so that a distal end of lumen 4 is adjacent an intended target site for agent 100 administration. As previously discussed, such delivery and positioning may be accomplished via an endoscope having a working channel (not shown). Imaging associated with the endoscope may assist in positioning. A user may then load one or more of the plurality of enclosures 31a-h of holster 30 with agent 100', if not loaded already. The user may then rotate holster 30 relative a remainder of housing 36 and lumen 4 so that one of enclosures 31a-31h aligns with first opening 32, thereby dropping agent 100' into chamber 33. Wedge 37 proceeds to apply a force onto agent 100', thereby separating/ crushing agent 100' into particles (not shown). Rotation of holster 30 may be by any suitable manner or mechanism, e.g., by hand or by mechanical, electrical, or pneumatic action. Said rotation may also be a measured rotation or a continuous rotation via a mechanical or electrical means. Because there is fluid communication between chamber 33 and lumen 4, via channel 35, said particles are delivered to lumen 4, and are propelled towards a distal end of lumen 4 via pressurized fluid. It is noted that pressurized fluid may be supplied to lumen 4, by a fluid source, at any time prior to, during, and after the rotation of holster 30.

It will be apparent to those skilled in the art that various modifications and variations can be made to the disclosed device without departing from the scope of the disclosure. Other embodiments of the disclosure will be apparent to those skilled in the art from consideration of the specification and practice of the invention disclosed herein. It is intended that the specification and examples be considered as exemplary only, while the scope of the invention for which protection is sought is defined by the appended set of claims.

## Claims

1. A medical device (1"') comprising:
a fluid source (7) providing a pressurized fluid;
a housing (36) defining at least one enclosure for storing an agent (100') in a first form;
a lumen (4) defined by the medical device;
a force applicator (37) within the housing and adjacent the enclosure; and
a first channel or a port branched from the lumen and feeding the pressurized fluid towards the force applicator, wherein the force applicator defines a surface for applying a force to the agent to separate the agent into particles smaller than a size of the first form in response to the pressurized fluid actuating the force applicator;
wherein the lumen (4) is adapted for receiving the particles from the force applicator and for receiving the pressurized fluid from the fluid source to propel the particles through the lumen.

2. The medical device of claim 1, wherein the force applicator is provided in the form of a wedge.

3. The medical device of claim 1 or claim 2, wherein the agent in the first form is provided in the form of pellets.

4. The medical device of any one of claims 1-3, wherein the housing includes a holster (30) defining a plurality of enclosures (31a - 31h) for storing the agent, wherein the holster is rotatable relative to other portions of the housing and the lumen.

5. The medical device of claim 4, wherein the housing further includes a chamber (33) below the holster, and a second channel (35) between the chamber and the lumen so that there is fluid communication between the chamber and the lumen.

6. The medical device of claim 5, wherein the first channel is branched from the lumen at a point that is proximal to the second channel.

7. The medical device of any one of claims 1-6, further comprising a drive mechanism (13) for moving the agent toward the force applicator.

8. The medical device of claim 7 in combination with claim 5 or claim 6, wherein the drive mechanism is configured to effect a rotation of the holster so that one of the plurality of enclosures aligns with the chamber, thereby delivering the agent from one of the enclosures to the chamber.

9. The medical device of any one of claims 5-8 in combination with claim 2, wherein the wedge obtrudes into the chamber, and the wedge is configured to separate the agent in the chamber into particles.

10. The medical device of any one of claims 5-9, wherein the chamber defines a first opening (32) that is adjacent to the holster, and a narrower, second opening (34) leading to the second channel which leads to the lumen.

11. The medical device of claim 10, configured such that the agent is inhibited from falling into the second channel and being delivered to the lumen prior to being separated into the particles smaller than the size of the first form, in that the second opening and the second channel are narrower than a width or cross-sectional size of the agent in the first form.

12. The medical device of any one of claims 4-11, configured such that rotation of the holster is caused by pneumatic action.

13. The medial device of any one of claims 1-12, wherein the pressurized gas is CO₂.

14. The medical device of any one of claims 1-13, further comprising a spring (38) that is configured to actuate the force applicator for applying the force onto the agent.

15. The medical device of any one of claims 1-14, wherein the particles smaller than the size of the first form comprise a powder of loose particles.

## Patentansprüche

1. Medizinische Vorrichtung (1"'), aufweisend:
eine Fluidquelle (7), die ein unter Druck stehendes Fluid bereitstellt;
ein Gehäuse (36), das mindestens eine Einfassung zum Speichern eines Wirkstoffs (100') in einer ersten Form definiert;
ein Lumen (4), das durch die medizinische Vorrichtung definiert ist;
einen Kraftapplikator (37) in dem Gehäuse und benachbart zur Einfassung; und
einen ersten Kanal oder einen Port, der vom Lumen abzweigt und das unter Druck stehende Fluid zu dem Kraftapplikator hin führt, wobei der Kraftapplikator eine Oberfläche zum Applizieren einer Kraft auf den Wirkstoff bildet, um den Wirkstoff in Teilchen zu trennen, die kleiner sind als eine Größe der ersten Form, in Reaktion darauf, dass das unter Druck stehende Fluid den Kraftapplikator betätigt;
wobei das Lumen (4) ausgebildet ist zum Aufnehmen der Teilchen von dem Kraftapplikator und zum Aufnehmen des unter Druck stehenden Fluids von der Fluidquelle, um die Teilchen durch das Lumen hindurch vorwärtszutreiben.

2. Medizinische Vorrichtung nach Anspruch 1, wobei der Kraftapplikator in Form eines Keils bereitgestellt ist.

3. Medizinische Vorrichtung nach Anspruch 1 oder Anspruch 2, wobei der Wirkstoff in der ersten Form in Form von Pellets bereitgestellt ist.

4. Medizinische Vorrichtung nach irgendeinem der Ansprüche 1-3, wobei das Gehäuse ein Holster (30) aufweist, das eine Mehrzahl von Einfassungen (31a - 31h) zum Speichern des Wirkstoffs definiert, wobei das Holster relativ zu anderen Abschnitten des Gehäuses und des Lumens drehbar ist.

5. Medizinische Vorrichtung nach Anspruch 4, wobei das Gehäuse ferner eine Kammer (33) unter dem Holster umfasst, und einen zweiten Kanal (35) zwischen der Kammer und dem Lumen, so dass Fluidkommunikation zwischen der Kammer und dem Lumen besteht.

6. Medizinische Vorrichtung nach Anspruch 5, wobei der erste Kanal vom Lumen an einem Punkt abzweigt, der proximal zum zweiten Kanal ist.

7. Medizinische Vorrichtung nach irgendeinem der Ansprüche 1-6, ferner umfassend einen Antriebsmechanismus (13) zum Bewegen des Wirkstoffs zu dem Kraftapplikator hin.

8. Medizinische Vorrichtung nach Anspruch 7 in Kombination mit Anspruch 5 oder Anspruch 6, wobei der Antriebsmechanismus so konfiguriert ist, dass er eine Drehung des Holsters bewirkt, so dass sich eine der Mehrzahl von Einfassungen zur Kammer ausrichtet, um dadurch den Wirkstoff aus einer der Einfassungen an die Kammer abzugeben.

9. Medizinische Vorrichtung nach irgendeinem der Ansprüche 5-8 in Kombination mit Anspruch 2, wobei der Keil in die Kammer hineinstößt und der Keil so konfiguriert ist, dass er den Wirkstoff in der Kammer in Teilchen trennt.

10. Medizinische Vorrichtung nach irgendeinem der Ansprüche 5-9, wobei die Kammer eine erste Öffnung (32) definiert, die benachbart zum Holster ist, und eine schmalere, zweite Öffnung (34), die zum zweiten Kanal führt, der zu dem Lumen führt.

11. Medizinische Vorrichtung nach Anspruch 10, die so konfiguriert ist, dass der Wirkstoff daran gehindert wird, in den zweiten Kanal zu fallen und zum Lumen abgegeben zu werden, bevor er in die Teilchen getrennt wird, die kleiner sind als die Größe der ersten Form, dadurch dass die zweite Öffnung und der zweite Kanal schmaler sind als eine Breite oder Querschnittsgröße des Wirkstoffs in der ersten Form.

12. Medizinische Vorrichtung nach irgendeinem der Ansprüche 4-11, die so konfiguriert ist, dass Drehung des Holsters durch pneumatische Wirkung verursacht wird.

13. Medizinische Vorrichtung nach irgendeinem der Ansprüche 1-12, wobei das unter Druck stehende Gas CO₂ ist.

14. Medizinische Vorrichtung nach irgendeinem der Ansprüche 1-13, ferner umfassend eine Feder (38), die so konfiguriert ist, dass sie den Kraftapplikator zum Applizieren der Kraft auf den Wirkstoff betätigt.

15. Medizinische Vorrichtung nach irgendeinem der Ansprüche 1-14, wobei die Teilchen, die kleiner sind als die Größe der ersten Form, ein Pulver aus losen Teilchen umfassen.

## Revendications

1. Dispositif médical (1"') comprenant :
une source de fluide (7) fournissant un fluide pressurisé ;
un boîtier (36) définissant au moins une enceinte pour stocker un agent (100') sous une première forme ;
une lumière (4) définie par le dispositif médical ;
un applicateur de force (37) à l'intérieur du boîtier et adjacent à l'enceinte ; et
un premier canal ou un orifice ramifié à partir de la lumière et introduisant le fluide pressurisé vers l'applicateur de force, dans lequel l'applicateur de force définit une surface pour appliquer une force à l'agent pour séparer l'agent en particules plus petites que la taille de la première forme en réponse au fluide pressurisé actionnant l'applicateur de force ;
dans lequel la lumière (4) est adaptée pour recevoir les particules provenant de l'applicateur de force et pour recevoir le fluide pressurisé provenant de la source de fluide pour propulser les particules à travers la lumière.

2. Dispositif médical selon la revendication 1, dans lequel l'applicateur de force se présente sous la forme d'un coin.

3. Dispositif médical selon la revendication 1 ou la revendication 2, dans lequel l'agent sous la première forme se présente sous la forme de pastilles.

4. Dispositif médical selon l'une quelconque des revendications 1 à 3, dans lequel le boîtier inclut un étui (30) définissant une pluralité d'enceintes (31a-31h) pour stocker l'agent, dans lequel l'étui peut tourner par rapport à d'autres parties du boîtier et la lumière.

5. Dispositif médical selon la revendication 4, dans lequel le boîtier inclut en outre une chambre (33) sous l'étui, et un deuxième canal (35) entre la chambre et la lumière de façon qu'il y ait une communication de fluide entre la chambre et la lumière.

6. Dispositif médical selon la revendication 5, dans lequel le premier canal est ramifié à partir de la lumière à un point qui est proximal au deuxième canal.

7. Dispositif médical selon l'une quelconque des revendications 1 à 6, comprenant en outre un mécanisme d'entraînement (13) pour déplacer l'agent vers l'applicateur de force.

8. Dispositif médical selon la revendication 7 en combinaison avec la revendication 5 ou la revendication 6, dans lequel le mécanisme d'entraînement est configuré pour effectuer une rotation de l'étui de façon que l'une parmi la pluralité d'enceintes s'aligne avec la chambre, en délivrant ainsi l'agent à la chambre depuis l'une des enceintes.

9. Dispositif médical selon l'une quelconque des revendications 5 à 8 en combinaison avec la revendication 2, dans lequel le coin empiète dans la chambre, et le coin est configuré pour séparer l'agent dans la chambre en particules.

10. Dispositif médical selon l'une quelconque des revendications 5 à 9, dans lequel la chambre définit une première ouverture (32) qui est adjacente à l'étui, et une deuxième ouverture plus étroite (34) conduisant au deuxième canal qui conduit à la lumière.

11. Dispositif médical selon la revendication 10, configuré de façon que l'agent soit empêché de tomber dans le deuxième canal et d'être délivré à la lumière avant d'être séparé en les particules plus petites que la taille de la première forme, étant donné que la deuxième ouverture et le deuxième canal sont plus étroits que la largeur ou la taille en coupe transversale de l'agent sous la première forme.

12. Dispositif médical selon l'une quelconque des revendications 4 à 11, configuré de façon que la rotation de l'étui soit provoquée par une action pneumatique.

13. Dispositif médical selon l'une quelconque des revendications 1 à 12, dans lequel le gaz pressurisé est du CO₂.

14. Dispositif médical selon l'une quelconque des revendications 1 à 13, comprenant en outre un ressort (38) qui est configuré pour actionner l'applicateur de force pour appliquer la force sur l'agent.

15. Dispositif médical selon l'une quelconque des revendications 1 à 14, dans lequel les particules plus petites que la taille de la première forme comprennent une poudre de particules libres.
